# FASCICULE DE BREVET EUROPEEN

(11) **EP 0 566 488 B1**
(45) Date de publication et mention de la délivrance du brevet: **04.09.1996**
(21) Numéro de dépôt: 93400977.0
(22) Date de dépôt: 15.04.1993
(51) Int. Cl.: A61M 16/18

(54) **Procédé et dispositif d'élaboration d'un mélange gazeux anesthésique**
Verfahren und Vorrichtung zur Herstellung eines anästhetischen Gasgemisches
Method and apparatus for preparing an anaesthetic gas mixture

(30) Priorité: 15.04.1992 FR 9204605
(43) Date de publication de la demande: 20.10.1993
(73) Titulaire: TAEMA, F-92182 Antony Cédex (FR)
(72) Inventeur: Bloch, Nicolas, F-75014 Paris (FR); Ruton, Stéphane, F-78220 Viroflay (FR); Monnot, André, F-78310 Elancourt-Maurepas (FR); Renaudin, Marie-Hélène, F-75015 Paris (FR)
(74) Mandataire: Le Moenner, Gabriel

(56) Documents cités:
- EP-A- 0 153 988
- EP-A- 0 231 513
- EP-A- 0 361 134
- EP-A- 0 449 545

## Description

La présente invention concerne les procédés d'élaboration d'un mélange gazeux anesthésique contenant de l'oxygène, un gaz additionnel, typiquement du protoxyde d'azote ou de l'air, et un agent anesthésique halogéné en phase gazeuse, comme décrit dans EP-A-0 231 513 et EP-A-0 361 134.

Les procédés actuellement utilisés mettent en oeuvre des dispositifs spécifiques pour chaque gaz vecteur (débitmètre de type rotamètre) et pour chaque agent anesthésique (conteneurs où l'on verse l'agent anesthésique liquide et où on fait débiter le mélange d'oxygène et de gaz additionnel préalablement réglé manuellement pour extraire le mélange gazeux anesthésique). Ce procédé présente de nombreux inconvénients avérés, en particulier une faible précision, une absence de contrôle du débit et de la composition du mélange gazeux anesthésique, des risques de confusion entre les agents anesthésiques présents dans le conteneur de formation du mélange gazeux anesthésique et de pollution de la salle d'opération lors du remplissage du récipient, une grande sensibilité à la température et à la pression environnantes, et une mauvaise ergonomie.

La présente invention a pour objet de proposer un procédé simple, fiable, ergonomique, permettant d'utiliser un dispositif unique pour les différents gaz et agents anesthésiques, garantissant une précision et des conditions de sécurité accrues grâce notamment à un contrôle automatique du débit et de la composition du mélange gazeux anesthésique exempt d'air parasite.

Pour ce faire, selon une caractéristique de l'invention, le procédé comporte les étapes de générer, à partir de sources d'oxygène et d'au moins un gaz additionnel, un mélange gazeux d'oxygène et de gaz additionnel, de prélever un débit d'agent anesthésique en phase liquide, par pressurisation avec de l'oxygène d'un conteneur, qui est avantageusement la bouteille d'origine de l'agent anesthésique, de le vaporiser par chauffage, et de mélanger la vapeur pure d'agent anesthésique ainsi obtenue avec le mélange pré-établi d'oxygène et de gaz additionnel.

La présente invention a également pour objet un dispositif d'élaboration d'un mélange gazeux anesthésique contenant de l'oxygène, un gaz additionnel, et un agent anesthésique halogéné en phase vapeur à partir d'une source d'oxygène, d'une source de gaz additionnel et d'un volume d'agent anesthésique en phase liquide contenu dans un conteneur, une ligne de transfert, incluant un vaporiseur thermique, de l'agent anesthésique s'étendant depuis le volume d'agent anesthésique dans le conteneur et débouchant dans un poste de mélange dans lequel débouche également une ligne d'amenée d'un mélange d'oxygène et de gaz additionnel reliée aux sources d'oxygène et de gaz additionnel, caractérisé en ce qu'il comporte une ligne de pressurisation, provenant de la source d'oxygène et reliée hermétiquement au conteneur.

Le procédé et le dispositif selon l'invention permettent notamment d'éviter l'introduction, avec l'agent anesthésique vaporisé d'air parasite, et donc d'azote, peu soluble dans le sang, et par conséquent d'éviter l'effet indésirable dit "effet deuxième gaz" en anesthésie.

D'autres caractéristiques et avantages de la présente invention ressortiront de la description suivante d'un mode de réalisation, donnée à titre illustratif mais nullement limitatif, faite en relation avec les dessins annexés, sur lesquels :
- la figure 1 représente schématiquement un premier mode de réalisation d'un dispositif selon l'invention ; et
- la figure 2 représente schématiquement un deuxième mode de réalisation d'un dispositif selon l'invention.

Dans la description qui va suivre et sur les dessins, les éléments identiques ou analogues portent les mêmes références.

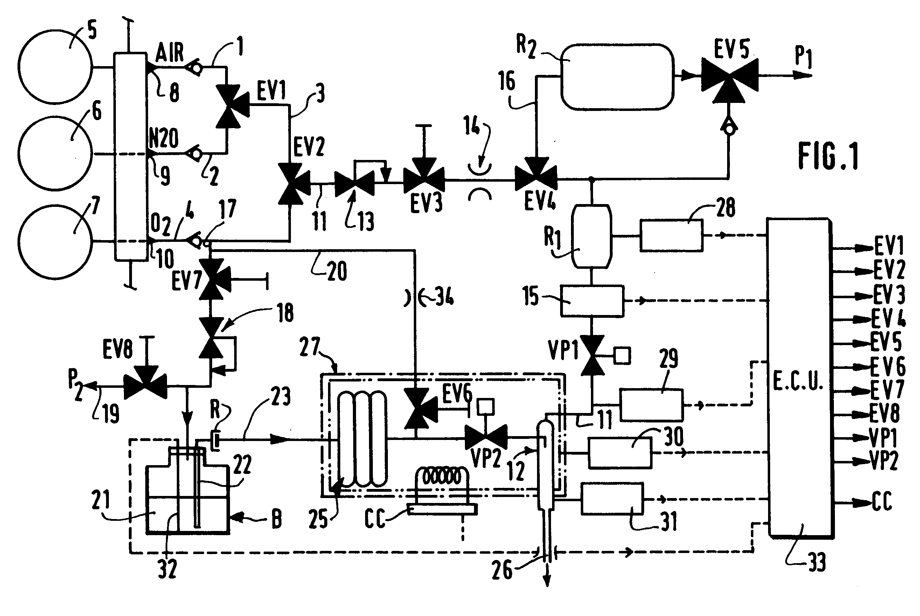

Le dispositif représenté sur la figure 1 comporte deux lignes 1 et 2 reliées à une électrovanne à trois voies EV₁ d'où part un tronçon de ligne 3 relié à une électrovanne EV₂ également reliée à une ligne 4. Les lignes 1, 2 et 4 sont reliées respectivement à des sources d'air 5, de protoxyde d'azote 6, d'oxygène 7 sous pression, par exemple via des prises murales correspondantes 8, 9 et 10. En sortie de l'électrovanne EV₂ s'étend une ligne 11 débouchant, comme on le verra plus avant, dans un poste de mélange 12 et comprenant, successivement, un détendeur 13, une électrovanne EV₃, un orifice calibré laminaire 14, un réservoir R₁, un capteur de débit 15, et une électrovanne proportionnelle de régulation de débit VP₁. De préférence, on prévoira, en amont du réservoir R₁, une boucle 16 émanant d'une électrovanne EV₄ dans la ligne 11 et incluant un second réservoir R₂ de plus grande capacité que le réservoir R₁ et une électrovanne aval EV₅ reliée également à une ligne de purge P₁.

Selon un aspect de l'invention, de la ligne d'oxygène 4 part une ligne de pressurisation 17 débouchant dans un conteneur hermétique B et comportant successivement, une électrovanne EV₇, un détendeur 18 et une dérivation de purge 19 contenant une électrovanne EV₈. Le conteneur B est une bouteille contenant d'origine un volume liquide 21 d'un agent anesthésique halogéné, tel que l'halothane, l'enflurane, l'isoflurane, le sévoflurane ou le desflurane. Dans la bouteille B est monté, traversant de façon hermétique le bouchon obturateur de la bouteille, un tube plongeur 22 relié, par un raccord démontable R, à une ligne de transfert 23 comportant, successivement, une partie 25 formant serpentin de vaporisation, et une vanne motorisée de réglage fin de débit VP₂, et débouchant coaxialement dans le poste de mélange 12 qui est relié, en aval, à un circuit 26 d'administration de mélange gazeux anesthésique à un patient. Une ligne de purge 20, comportant une restriction calibrée 34 et une électrovanne EV₆, relie la partie amont de la ligne de pressurisation 17 et un point de la ligne de transfert 23 entre le serpentin 25 et la vanne motorisée VP₂ pour refouler, dans le conteneur B, en fin d'utilisation, le volume d'agent anesthésique dans le serpentin 25 et la partie amont de la ligne 23 afin de purger ces derniers en vue d'une utilisation ultérieure avec un autre agent anesthésique. Le serpentin 25, les vannes VP₂ et EV₆ et le poste de mélange 12 sont disposés dans une enceinte thermostatée 27 chauffée par un circuit électrique de chauffage CC.

Le dispositif comporte en outre plusieurs détecteurs de paramètres significatifs pour son contrôle, à savoir un capteur 28 de la pression de mélange gazeux régnant dans le réservoir R₁, un analyseur 29 de la teneur en oxygène dans le mélange gazeux acheminé par la ligne 11, un capteur 30 de la température dans l'enceinte 27, un analyseur 31 de la concentration en agent anesthésique halogéné dans le mélange gazeux anesthésique délivré par le circuit 26, et un détecteur 32 de niveau de l'agent anesthésique en phase liquide dans sa bouteille 18. Ces différents détecteurs fournissent des signaux de contrôle à une unité centrale de commande électronique 33, du type à microprocesseur, élaborant et fournissant en sorties (partie droite de la figure unique) des signaux de commande et de surveillance aux différentes électrovannes.

Le fonctionnement du dispositif est le suivant :

### - fonctionnement du mélangeur :

L'utilisateur fixe, par entrée de données dans l'unité de commande 33, la nature du mélange gazeux (O₂/N₂O ou O₂/air), le débit total de mélange gazeux ou gaz frais délivré par la ligne 11, et la concentration en oxygène désirée dans ce mélange gazeux. L'électrovanne EV₂, pilotée par l'unité centrale 33, est commutée alternativement entre les lignes d'amenée de gaz 3 et 4 pour obtenir, dans la ligne 11, la concentration en oxygène requise, qui ne doit pas être inférieure à 25 %. La période et le rapport cyclique de battement de l'électrovanne EV₂ sont asservies à la valeur théorique du débit pré-réglé ainsi qu'à la mesure de la concentration en oxygène fournie par le capteur 29. Le débit de vidange des réservoirs R₁, R₂ doit être égal au débit de mélange de gaz frais qui leur est adressé et qui correspond à une consigne fixée par l'utilisateur. Le capteur de débit 15 et la vanne de réglage VP₁ contrôlent en continu la valeur du débit global du mélange gazeux de gaz frais fourni par rapport à la consigne. Le début ou l'arrêt de la phase de remplissage des réservoirs R₁, R₂ est commandé par l'électrovanne EV₃ en fonction du signal fourni par le capteur de pression 28. Cet agencement permet de maintenir dans les réservoirs R₁ et R₂ une pression comprise entre un seuil minimal (typiquement Pₘᵢₙ = 1,3 x 10⁵ Pa abs) et un seuil maximal (typiquement Pₘₐₓ = 2,1 x 10⁵ Pa abs). Pendant la phase de remplissage, les électrovannes EV₁ et EV₂ sont ouvertes successivement pour maintenir la concentration en oxygène requise sus-mentionnée.

Pour que le temps de réponse du circuit d'obtention du mélange gazeux, lors d'une modification des valeurs de consigne, soit satisfaisant dans toute la gamme de débits de mélange gazeux de gaz frais envisagée (typiquement 0,2 l/mn à 20 l/mn), il est préférable d'utiliser les deux réservoirs R₁ et R₂ en série. Les électrovannes EV₄ et EV₅ permettent d'utiliser soit le réservoir R₁ seul (typiquement d'une capacité d'environ 0,2 litre) lorsque le débit de mélange gazeux est compris entre 0,2 l/mn et 2 l/mn, soit les deux réservoirs R₁ et R₂ en série (offrant ainsi un volume total d'environ 0,9 l) lorsque le débit de mélange gazeux de gaz frais est compris entre 2 l/mn et 20 l/mn. L'analyseur d'oxygène 29 permet de contrôler le fonctionnement du dispositif et d'ajuster le temps d'ouverture de l'électrovanne EV₂ si la concentration en oxygène mesurée par l'analyseur 29 s'écarte de la valeur de consigne.

### - fonctionnement de l'évaporateur :

L'utilisateur met en place la bouteille d'agent halogéné B et affiche dans l'unité de commande 33 les valeurs de consigne pour le débit total de mélange gazeux anesthésique et la concentration en agent halogéné dans ce dernier. Le dispositif de fixation de la bouteille permet d'identifier l'agent halogéné concerné. Le liquide dans la bouteille B est placé, par la ligne de pressurisation 17, sous une pression d'oxygène constante, typiquement de 2 x 10⁵ Pa abs, et s'écoule ainsi dans la ligne de transfert 23. De façon à optimiser l'échange thermique dans l'enceinte 27, le serpentin 25 est avantageusement constitué d'un tube métallique de faible diamètre et de grande longueur. Le débit de vapeur pure d'agent halogéné en sortie du serpentin 25 est contrôlé par la vanne de réglage VP₂ pilotée par un moteur électrique. Ce débit d'agent anesthésique vaporisé est ensuite introduit, dans le poste de mélange 12, dans le courant de mélange gazeux acheminé par la ligne 11 parallèlement à celui-ci de façon à obtenir une dilution homogène. La température dans l'enceinte 27 est maintenue à une valeur de l'ordre de 80°C de façon que la tension de vapeur de chaque agent anesthésique halogéné à cette température soit supérieure à 2 x 10⁵ Pa abs, pour éviter toute recondensation de liquide.

La vanne motorisée de réglage VP₂ permet d'obtenir des débits de vapeur pure d'agent halogéné allant de 0,2 ml/mn (soit une concentration d'agent halogéné de 0,1 % dans un débit de mélange gazeux frais de 0,2 l/mn) à 1000 ml/mn (soit une concentration d'agent halogéné de 5 % dans un débit de mélange gazeux frais de 20 l/mn). L'analyseur 31 mesure la concentration en agent anesthésique dans le mélange gazeux anesthésique et permet d'ajuster le réglage de la vanne motorisée de réglage VP₂ si la concentration mesurée s'écarte de la valeur de consigne introduite dans l'unité de commande 33. Les électrovannes EV₆, EV₇ et EV₈ sont utilisées pour dépressuriser et purger le circuit évaporateur lors d'un changement de bouteille.

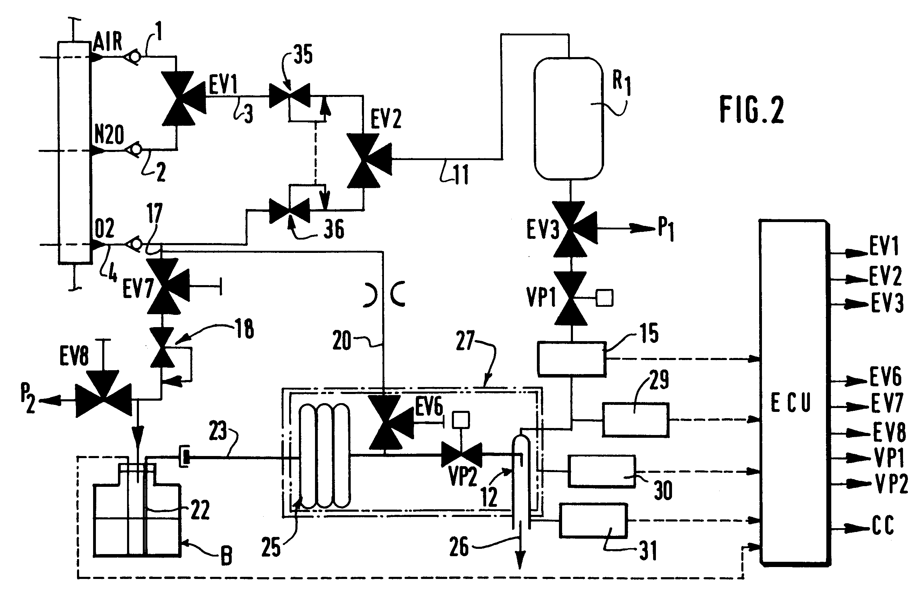

On a représenté sur la figure 2 un dispositif simplifié. Dans cette version, n'utilisant qu'un seul réservoir R₁, le détendeur 13, la restriction calibrée 14, les électrovannes EV₄ et EV₅ et le capteur de pression 28 sont supprimés. L'électrovanne EV₃, ici du type à trois voies, est disposée dans la ligne de transfert 11 entre le réservoir R₁ et la vanne de réglage VP₁, la troisième voie étant reliée à la ligne de purge P₁. Le capteur de débit 15 est disposé en aval de la vanne de réglage VP₁. Dans ce mode de réalisation, les lignes de gaz additionnel 3 et d'oxygène 4 comportent des détendeurs couplés 35, 36 permettant d'équilibrer les pressions en amont de l'électrovanne EV₂. L'établissement, par cette électrovanne EV₂, d'une liaison entre la ligne de transfert 11 et la ligne de gaz additionnel 3 ou la ligne d'oxygène 4 permet de fixer la concentration en oxygène dans le mélange gazeux frais. Si on appelle T_{O2} le temps de passage de l'oxygène au travers de l'électrovanne EV₂ et T_{gaz} le temps de passage du gaz additionnel au travers de cette électrovanne, et si l'on fixe la somme de T_{O2} et T_{gaz} à une valeur constante, la concentration en oxygène dans le melange gazeux frais est alors directement fonction du rapport T_{O2}/T_{gaz}. Dans ce mode de réalisation, les phases de remplissage et de vidange du réservoir R₁ ne sont plus séquentielles et ne dépendent plus de la pression dans le réservoir, le remplissage et la vidange de ce dernier s'effectuant en continu, la vanne de réglage VP₁ modulant le débit total de mélange en fonction du signal fourni par le capteur de débit 15. Dans une variante avantageuse (non représentée), de ce dernier mode de réalisation, la vanne de réglage VP₁ est reportée, dans la ligne de transfert 11, en amont du réservoir R₁ et le détendeur 18 est reporté, dans la ligne de pressurisation 17, en amont du point de départ de la ligne de purge 20.

Quoique la présente invention ait été décrite en relation avec un mode de réalisation particulier, elle ne s'en trouve pas limitée pour autant mais est au contraire susceptible de modifications et de variantes qui apparaîtront à l'homme de l'art.

## Revendications

1. Procédé d'élaboration d'un mélange gazeux anesthésique contenant de l'oxygène, un gaz additionnel et un agent anesthésique halogéné, caractérisé en ce qu'il comprend les étapes de générer, à partir d'une source d'oxygène (7) et d'au moins une source de gaz additionnel (5 ; 6), un mélange gazeux d'oxygène et de gaz additionnel, de prélever un débit d'agent anesthésique en phase liquide, par pressurisation avec de l'oxygène (17), d'un conteneur (B) contenant l'agent anesthésique, de le vaporiser par chauffage (25), et de mélanger la vapeur d'agent anesthésique avec le mélange pré-établi (11) d'oxygène et de gaz additionnel.

2. Procédé selon la revendication 1, caractérisé en ce que le conteneur (B) est la bouteille de l'agent anesthésique, et en ce que l'oxygène de pressurisation est régulé à partir de la source d'oxygène (7).

3. Procédé selon l'une des revendications précédentes, caractérisé en ce qu'il comprend les étapes de réguler le débit et la composition du mélange gazeux anesthésique (26).

4. Procédé selon la revendication 3, caractérisé en ce qu'il comprend l'étape de réguler (CC) la température de vaporisation de l'agent anesthésique.

5. Procédé selon l'une des revendications précédentes, caractérisé en ce que la concentration en oxygène dans le mélange d'oxygène et de gaz additionnel est modulée par fourniture séquentielle (EV₂) de l'oxygène et du gaz additionnel (EV₁).

6. Dispositif d'élaboration d'un mélange gazeux anesthésique contenant de l'oxygène, un gaz additionnel et un agent anesthésique halogéné à partir d'une source d'oxygène (7), d'une source de gaz additionnel (5 ; 6) et d'un volume (21) d'agent anesthésique en phase liquide dans un conteneur (B), comprenant une ligne (23), incluant un vaporiseur thermique (25), de transfert de l'agent anesthésique s'étendant depuis le volume (21) d'agent anesthésique dans le conteneur (B) et débouchant dans un poste de mélange (12) dans lequel débouche également une ligne (11) d'amenée d'un mélange d'oxygène et de gaz additionnel reliée aux sources d'oxygène et de gaz additionnel (7 ; 5 ; 6), caractérisé en ce qu'il comporte une ligne de pressurisation (17) provenant de la source d'oxygène (7) et reliée hermétiquement au conteneur d'agent anesthésique (B).

7. Dispositif selon la revendication 6, caractérisé en ce que la ligne de pressurisation (17) inclut des moyens de réglage de pression (18).

8. Dispositif selon l'une des revendications 6 et 7, caractérisé en ce que le conteneur (B) est la bouteille d'origine de l'agent anesthésique.

9. Dispositif selon l'une des revendications 6 à 8, caractérisé en ce que le vaporiseur est constitué d'un serpentin (25) disposé dans une enceinte thermostatée (27).

10. Dispositif selon l'une des revendications 6 à 9, caractérisé en 'ce que le poste de mélange (12) est disposé dans l'enceinte thermostatée (27).

11. Dispositif selon l'une des revendications 6 à 10, caractérisé en ce qu'il comporte des moyens (VP₁, EV₃) de réglage du débit et de la pression du mélange gazeux d'oxygène et de gaz additionnel.

12. Dispositif selon l'une des revendications 6 à 11, caractérisé en ce qu'il comporte des moyens (VP₂) de réglage du débit d'agent anesthésique pur vaporisé.

13. Dispositif selon la revendication 11 ou la revendication 12, caractérisé en ce que les différents moyens de réglage de débit (VP₂, VP₁, EV₃) sont pilotés par une unité de commande électronique (33).

14. Dispositif selon la revendication 13, caractérisé en ce qu'il comporte, dans la ligne (11) d'amenée du mélange gazeux d'oxygène et du gaz additionnel, un analyseur d'oxygène (29) relié à l'unité de commande électronique (33).

15. Dispositif selon la revendication 13 ou la revendication 14, caractérisé en ce qu'il comporte, en sortie du poste de mélange (12), un analyseur (31) de concentration d'agent anesthésique, relié à l'unité de commande électronique (33).

16. Dispositif selon l'une des revendications 6 à 15, caractérisé en ce qu'il comporte une ligne de purge (20) munie d'une électrovanne (EV₆), reliée à la source d'oxygène (7) et à la ligne (23) de transfert d'agent anesthésique (23) en aval du vaporiseur, (25).

## Claims

1. Process for the preparation of an anaesthetic gaseous mixture containing oxygen, an additional gas and a halogenated anaesthetic agent, characterized in that it comprises the stages of generating, from a source of oxygen (7) and at least one source of additional gas (5; 6), a gaseous mixture of oxygen and an additional gas, of withdrawing a flow of anaesthetic agent in the liquid phase, by pressurisation with oxygen (17), from a container (B) containing the anaesthetic agent, of vaporizing it by heating (25), and of mixing the vapour of the anaesthetic agent with the pre-established mixture (11) of oxygen and the additional gas.

2. Process according to claim 1, characterized in that the container (B) is a cylinder of the anaesthetic agent, and in that the pressurizing oxygen is regulated from the source of oxygen (7).

3. Process according to one of the preceding claims, characterized in that it comprises the stages of regulating the flow and composition of the anaesthetic gaseous mixture (26).

4. Process according to claim 3, characterized in that it comprises the stage (CC) of regulating the vaporization temperature of the anaesthetic agent.

5. Process according to one of the preceding claims, characterized in that the concentration of oxygen in the mixture of oxygen and the additional gas is modified by sequentially supplying oxygen (EV₂) and the additional gas (EV₁).

6. Device for preparing an anaesthetic gaseous mixture containing oxygen, an additional gas and a halogenated anaesthetic agent from a source of oxygen (7), a source of additional gas (5; 6) and a volume (21) of an anaesthetic agent in the liquid phase in a container (B), comprising a line (23), including a thermal vaporiser (25), for transferring the anaesthetic agent, extending from the volume (21) of anaesthetic agent in the container (B) and emerging in a mixing unit (12) into which a line (11) also emerges bringing a mixture of oxygen and additional gas connected to sources of oxygen and additional gas (7; 5; 6), characterized in that it comprises a pressurization line (17) coming from the source of oxygen (7) and connected hermetically to the container of anaesthetic agent (B).

7. Device according to claim 6, characterized in that the pressurization line (17) includes means for regulating the pressure (18).

8. Device according to either of claims 6 or 7, characterized in that the container (B) is an original cylinder of anaesthetic gas.

9. Device according to one of claims 6 to 8, characterized in that the vaporizer consists of a coil (25) disposed in a thermostatically controlled chamber (27).

10. Device according to one of claims 6 to 9, characterized in that the mixing unit (12) is disposed in the thermostatically controlled chamber (27).

11. Device according to one of claims 6 to 10, characterized in that it comprises means (VP₁, EV₃) for regulating the flow and the pressure of the gaseous mixture of oxygen and additional gas.

12. Device according to one of claims 6 to 11, characterized in that it comprises means (VP₂) for regulating the flow of vaporised pure anaesthetic agent.

13. Device according to claim 11 or claim 12, characterized in that the various means of regulating flow (VP₂, VP₁ and EV₃) are controlled by an electronic control unit (33).

14. Device according to claim 13, characterized in that it comprises, in the line (11) for bringing the gaseous mixture of oxygen and additional gas, an oxygen analyser (29) connected to the electronic control unit (33).

15. Device according to claim 13 or claim 14, characterized in that it comprises, at the outlet from the mixing unit (12), an analyser (31) for the concentration of anaesthetic agent, connected to the electronic control unit (33).

16. Device according to one of claims 6 to 15, characterized in that it comprises a purge line (20) provided with a solenoid valve (EV₆), connected to the source of oxygen (7) and to the line (23) for transferring the anaesthetic agent (23) downstream from the vaporizer (25).

## Patentansprüche

1. Verfahren zur Herstellung eines Sauerstoff, ein Zusatzgas und ein halogenhaltiges Betäubungsmittel enthaltenden, betäubenden Gasgemischs, gekennzeichnet durch die Schritte des Erzeugens eines gasförmigen Sauerstoff-Zusatzgas-Gemischs aus einer Sauerstoffquelle (7) und zumindest einer Zusatzgasquelle (5; 6), des Ausleitens einer Menge von in flüssiger Phase vorliegendem Betäubungsmittel aus einem das Betäubungsmittel enthaltenden Behälter (B) durch Druckbeaufschlagung mit Sauerstoff (17), des Verdampfens derselben durch Erwärmen (25), und des Mischens des Betäubungsmitteldampfs mit dem zuvor bereitgestellten Sauerstoff-Zusatzgas-Gemisch.

2. Verfahren nach Anspruch 1, dadurch gekennzeichnet, daß der Behälter (B) die Betäubungsmittelflasche ist, und daß der Sauerstoff zur Druckbeaufschlagung durch die Sauerstoffquelle (7) geregelt wird.

3. Verfahren nach einem der vorangehenden Ansprüche, gekennzeichnet durch die Schritte des Regelns der Menge und der Zusammensetzung des betäubenden Gasgemischs (26).

4. Verfahren nach Anspruch 3, gekennzeichnet durch den Schritt des Regelns (CC) der Temperatur zur Verdampfung des Betäubungsmittels.

5. Verfahren nach einem der vorangehenden Ansprüche, dadurch gekennzeichnet, daß die Sauerstoffkonzentration in dem Sauerstoff-Zusatzgas-Gemisch durch aufeinanderfolgende Zufuhr (EV₂) des Sauerstoffs und des Zusatzgases (EV₁) moduliert wird.

6. Vorrichtung zur Herstellung eines Sauerstoff, ein Zusatzgas und ein halogenhaltiges Betäubungsmittel enthaltenden, betäubenden Gasgemischs aus einer Sauerstoffquelle (7), einer Zusatzgasquelle (5, 6) und einem Volumen (21) eines in einem Behälter (B) in flüssiger Phase vorliegenden Betäubungsmittels, umfassend eine einen thermischen Verdampfer (25) enthaltende Betäubungsmittel-Transportleitung (23), die sich von dem Betäubungsmittelvolumen (21) in dem Behälter (B) ausgehend erstreckt und in eine Mischstation (12) mündet, in welche auch eine mit der Sauerstoffquelle (7) und der Zusatzgasquelle (5; 6) verbundene Leitung (11) zur Zufuhr eines Sauerstoff-Zusatzgas-Gemischs mündet, gekennzeichnet durch eine von der Sauerstoffgasquelle (7) wegführende und hermetisch mit dem Betäubungsmittelbehälter (B) verbundene Leitung (17) zur Druckbeaufschlagung.

7. Vorrichtung nach Anspruch 6, dadurch gekennzeichnet, daß die Leitung (17) zur Druckbeaufschlagung Druckregelmittel (17) enthält.

8. Vorrichtung nach einem der Ansprüche 6 und 7, dadurch gekennzeichnet, daß der Behälter (B) die Betäubungsmittel-Originalflasche ist.

9. Vorrichtung nach einem der Ansprüche 6 bis 8, dadurch gekennzeichnet, daß der Verdampfer aus einer in einer thermostabilisierten Hülle (27) angeordneten Schlange (25) besteht.

10. Vorrichtung nach einem der Ansprüche 6 bis 9, dadurch gekennzeichnet, daß die Mischstation (12) in der thermostabilisierten Hülle (27) angeordnet ist.

11. Vorrichtung nach einem der Ansprüche 6 bis 10, gekennzeichnet durch Mittel (VP₁, EV₃) zum Regeln der Menge und des Drucks des gasförmigen Sauerstoff-Zusatzgas-Gemischs.

12. Vorrichtung nach einem der Ansprüche 6 bis 11, gekennzeichnet durch Mittel (VP₂) zum Regeln der Menge reinen, verdampften Betäubungsmittels.

13. Vorrichtung nach Anspruch 11 oder 12, dadurch gekennzeichnet, daß die verschiedenen Mengenregelmittel (VP₂, VP₁, EV₃) durch eine elektronische Steuereinheit (33) gesteuert werden.

14. Vorrichtung nach Anspruch 13, gekennzeichnet durch einen in der Leitung (11) zur Zufuhr des Sauerstoff-Zusatzgas-Gemischs angeordneten, mit der elektronischen Steuereinheit (33) verbundenen Sauerstoffanalysator (29).

15. Vorrichtung nach Anspruch 13 oder 14, gekennzeichnet durch einen am Ausgang der Mischstation (12) angeordneten, mit der elektronischen Steuereinheit (33) verbundenen Betäubungsmittelkonzentrationsanalysator (31).

16. Vorrichtung nach einem der Ansprüche 6 bis 15, gekennzeichnet durch eine mit einem Elektroventil (EV₆) versehene, mit der Sauerstoffquelle (7) und stromab des Verdampfers (25) mit der Betäubungsmittel-Transportleitung (23) verbundene Auslaßleitung (20).
